# EUROPEAN PATENT APPLICATION

(11) **EP 4 342 509 A1**
(43) Date of publication of application: **27.03.2024**
(21) Application number: 23197666.3
(22) Date of filing: 15.09.2023
(51) Int. Cl.: A61M 5/31, A61M 5/315

(54) **TREATMENT OF DUAL CHAMBER SYRINGE AND METHODS OF USE THEREOF**

(30) Priority: 20.09.2022 US 202263408126 P
(71) Applicant: DALI MEDICAL DEVICES LTD., IL-Yavne 8122503 (IL)
(72) Inventor: KEENAN, Guy, 69710 Tel Aviv (IL); CARMEL, Ehoud, 5591618 Ganei Tikva (IL); SARKOROV, Dmitri, Rosh HaAyin (IL); RADAY, Lior, Kibbutz Bror-Hail (IL)
(74) Representative: Betten & Resch

(57) **Abstract**

A medical container, comprising a barrel having an inner surface partially treated with a friction reducing agent, the barrel having at least one substance chamber and wherein the at least one substance chamber defines a portion of the inner surface, which is not treated with the friction reducing agent.

## Description

### FIELD OF THE INVENTION

The present invention generally relates to treatment of a dual chamber syringe, and more specifically to treatment of a pre-fillable dual chamber syringe and methods of use thereof for reconstitution and injection of a medicament.

### BACKGROUND OF THE INVENTION

Pre-fillable dual chamber syringes are known in the art for separately containing several different substances, such as a powder/liquid medicament preparation and a solvent in different chambers of the syringe.

It is also known that pre-fillable dual chamber syringes preferably include a syringe barrel with several pistons, which are slidably sealingly disposed therewithin and divide the syringe barrel into several separate chambers, whereas one of the chambers contains a powder medicament and another contains a solvent. Alternatively, both chambers may include liquids that are stored separately and that shall be mixed only at the time of injection.

The pre-fillable dual chamber syringes also include a plunger rod, which is operative to engage one of the pistons. Upon displacement of the plunger rod relative to the syringe barrel, the pistons are advanced within the syringe barrel and permit reconstitution of the medicament by mixing the powder medicament with the solvent using a bypass formed in the syringe barrel.

It is known that a substantial friction force is created between the pistons and the pre-fillable dual chamber syringe, which the user needs to overcome during displacement of the plunger rod relative to the pre-fillable dual chamber syringe.

It is additionally known that one of the methods to reduce this friction force is treatment of the inner surface of the pre-fillable dual syringe with a lubricant, however oftentimes when one of the substances contained in the pre-fillable dual syringe contacts the lubricant, the lubricant is mixed into the substance and may cause either degradation of the substance due to contact with the lubricant or may cause a phenomenon of agglomeration, i.e., increase in the mean particle size of the substance. Agglomeration is known to reduce the dissolution rate between the two substances and thus has a negative effect on the efficacy of the medicament.

### SUMMARY OF THE INVENTION

The present invention seeks to provide an improved medical container.
There is thus provided in accordance with an embodiment of the present invention or a combination of embodiments thereof a medical container, comprising a barrel having an inner surface partially treated with a friction reducing agent; the barrel having at least one substance chamber and wherein the at least one substance chamber defines a portion of the inner surface, which is not treated with the friction reducing agent.

Preferably, the at least one substance chamber defines two substance chambers and at least one of the two substance chambers defines the portion of the inner surface, which is not treated with the friction reducing agent. Further preferably, wherein a drug preparation is configured to be contained within the at least one substance chamber confined by a piston, and the inner surface defined by the at least one substance chamber is not treated with friction reducing agent to avoid contact between the drug preparation and the friction reducing agent, and wherein the remainder of the inner surface of the barrel is treated with friction reducing agent.

Still further preferably, wherein a drug preparation is configured to be contained within the at least one substance chamber confined by a piston, and the inner surface defined by the at least one substance chamber is not treated with friction reducing agent to avoid contact between the drug preparation and the friction reducing agent, and wherein the remainder of the inner surface of the barrel is treated with friction reducing agent.

Yet further preferably, the friction reducing agent is a lubricant. Alternatively, the friction reducing agent is a coating substance.

Preferably, partial treatment of the inner surface of the barrel with friction reducing agent provides both for reduced friction forces between the barrel and the piston and for avoiding damage of the drug preparation during storage, due to lack of contact between the treated area of the barrel and the drug preparation.

In accordance with an embodiment of the present invention, the barrel having a forward end and a rearward end; a forward stopper, a first piston and a second piston rearwardly spaced from the first piston; the first piston and the second piston are configured to be slidably displaceable relative to the barrel; a first chamber is defined between the forward stopper and the first piston; a second chamber is defined between the first piston and the second piston; and wherein the inner surface is partially treated with a friction reducing agent, such that in storage the inner surface defined by at least one of the first chamber and the second chamber is not treated with the friction reducing agent.

Preferably, at least one bypass protrusion arranged along the longitudinal extent of the syringe barrel. Further preferably, a plunger rod is configured to be partially inserted into the barrel and slidably displace at least one of the first and second pistons relative to the barrel. Still further preferably, a drug preparation is confined within the first chamber and a solvent is confined within the second chamber and upon longitudinal displacement of the first and second pistons relative to the barrel, the drug preparation and the solvent are configured for reconstitution and subsequent ejection from the barrel. Yet further preferably, the inner surface that contains the bypass protrusion and adjacent to the bypass protrusion is not treated with friction reducing agent.

In accordance with an embodiment of the present invention, the inner surface of the barrel is only treated with friction reducing agent in the region where the first piston is configured to be seated in storage and axially rearwardly therefrom and in the region where the forward stopper is configured to be seated in storage and axially forwardly therefrom.

Preferably, the inner surface of the barrel defined between the forward stopper and the first piston is not treated with friction reducing agent, thereby preventing contact between a drug preparation and between the friction reducing agent in storage. Further preferably, partial treatment of the inner surface of the barrel with friction reducing agent is configured to reduce friction forces that are created during displacement of the plunger rod and the pistons relative to the barrel.

Still further preferably, partial treatment of the inner surface of the barrel with friction reducing agent provides both for reduced friction forces and avoiding damage of the drug preparation during storage, due to lack of contact between the treated area and the drug preparation.

### BRIEF DESCRIPTION OF THE DRAWINGS

The present invention will be understood and appreciated more fully from the following detailed description, taken in conjunction with the drawings in which:
Figs. 1A and 1B are respectively simplified pictorial view and exploded view of a dual chamber syringe constructed and operative in accordance with an embodiment of the present invention;
Figs. 2A and 2B are respectively a simplified perspective view and planar side view of a plunger rod inner portion forming part of the dual chamber syringe of Figs. 1A & 1B;
Figs. 3A, 3B, 3C, 3D and 3E are respectively a simplified perspective view, two simplified plan side views and two simplified sectional views taken along lines D - D and E - E in Fig. 3C of a plunger rod outer portion forming part of the dual chamber syringe of Figs. 1A & 1B;
Figs. 4A, 4B and 4C are simplified drawings of the dual chamber syringe of Figs. 1A - 3E in a storage operative orientation, including respectively a simplified planar view, a partially cut-out perspective view and a simplified sectional view taken along lines C - C in Fig. 4A;
Figs. 5A, 5B and 5C are simplified drawings of the dual chamber syringe of Figs. 1A - 3E in a pre-reconstitution operative orientation, including respectively a simplified planar view, a partially cut-out perspective view and a simplified sectional view taken along lines C - C in Fig. 5A;
Figs. 6A, 6B and 6C are simplified drawings of the dual chamber syringe of Figs. 1A - 3E in a medicament reconstitution operative orientation, including respectively a simplified perspective view, a partially cut-out perspective view and a simplified sectional view taken along lines C - C in Fig. 6A;
Figs. 7A, 7B and 7C are simplified drawings of the dual chamber syringe of Figs. 1A - 3E in an end of medicament reconstitution operative orientation, including respectively a simplified perspective view, a partially cut-out perspective view and a simplified sectional view taken along lines C - C in Fig. 7A;
Figs. 8A, 8B and 8C are simplified drawings of the dual chamber syringe of Figs. 1A - 3E in an end of injection operative orientation, including respectively a simplified perspective view, a partially cut-out perspective view and a simplified sectional view taken along lines C - C in Fig. 8A.

### DESCRIPTION OF EMBODIMENTS

The principles, uses and implementations of the teachings herein may be better understood with reference to the accompanying description and figures. Upon perusal of the description and figures present herein, one skilled in the art is able to implement the invention without undue effort or experimentation.

Before explaining at least one embodiment of the invention in detail, it is to be understood that the invention is not limited in its applications to the details of construction and the arrangement of the components and/or methods set forth in the following description and/or illustrated in the drawings and/or the Examples. The invention can be implemented with other embodiments and can be practiced or carried out in various ways. It is also understood that the phraseology and terminology employed herein is for descriptive purpose and should not be regarded as limiting.

Some embodiments of the invention are described herein with reference to the accompanying figures. The description, together with the figures, makes apparent to a person having ordinary skill in the art how some embodiments of the invention may be practiced. The figures are for the purpose of illustrative discussion and no attempt is made to show structural details of an embodiment in more detail than is necessary for a fundamental understanding of the invention. For the sake of clarity, some objects depicted in the figures are not to scale.

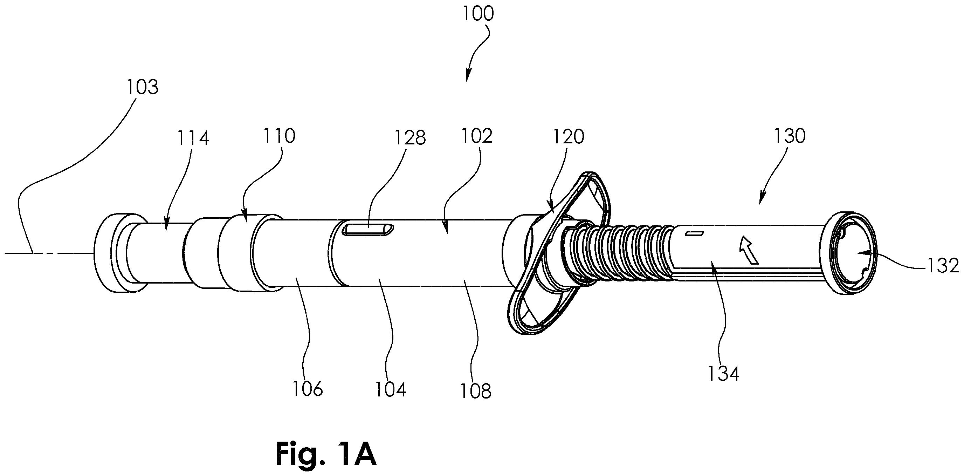

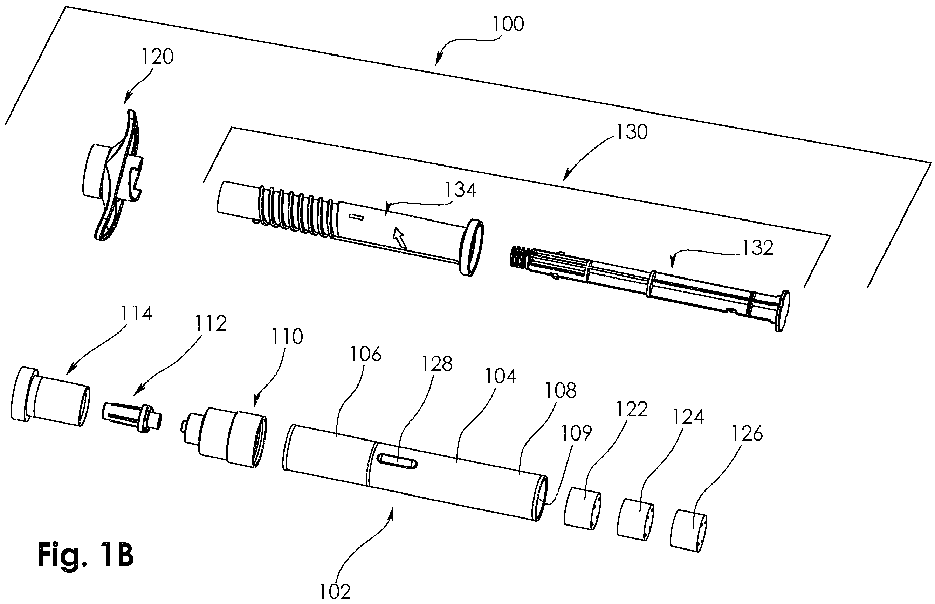

Reference is now made to Figs. 1A and 1B, which are respectively simplified pictorial view and exploded view of a dual chamber syringe constructed and operative in accordance with an embodiment of the present invention.

As seen in Figs. 1A & 1B, a dual chamber syringe 100 preferably includes a syringe assembly 102 arranged along a longitudinal axis 103. The syringe assembly 102 has a syringe barrel 104 having a forward end 106 and a rearward end 108. The syringe barrel has an inner surface 109. A luer lock element 110 is preferably fixedly and sealingly connected to the proximal end 106 of the syringe barrel 104. An inner cap element 112, which is adapted to seal and protect a portion of the luer lock element 110, and an outer cap 114 are preferably removably coupled to a forward end of the luer lock element 110. It is noted that the luer lock element 110, the inner cap element 112 and the outer cap 114 are preferably similar to the same elements described in U.S. Patent Application Serial No. 16/994,596, the disclosure of which is hereby incorporated by reference in its entirety.

It is also seen in Figs. 1A & 1B that a finger grip 120 is fixedly connected to the rearward end 108 of the syringe barrel 104. The syringe assembly 102 also preferably includes three pistons, namely a forward piston 122, which is also referred to hereinbelow as a forward stopper, an intermediate piston 124, which is also referred to hereinbelow as a first piston and a rearward piston 126, which is also referred to hereinbelow as a second piston. The forward piston 122, the intermediate piston 124 and the rearward piston 126 are contained within the syringe barrel 104 and are adapted for slidable axial displacement relative to the syringe barrel 104. It is appreciated that a first substance, such as a drug preparation is preferably confined between one pair of the pistons and a second substance, such as a solvent is preferably confined between another pair of the pistons and upon appropriate longitudinal displacement of the pistons, the two substances are configured for reconstitution and subsequent ejection, as described in detail hereinbelow.

It is noted that alternatively only two pistons may be contained within the syringe barrel 104, whereas in storage the first substance is sealed in this case by a forward stopper that is releasable from the syringe barrel 104 after reconstitution. Specifically, in accordance with this embodiment, the first substance, such as a drug preparation is preferably confined between the forward stopper 122 and the first piston 124 and a second substance, such as a solvent is preferably confined between the first piston 124 and the second piston 126. In accordance with this alternative embodiment, the luer lock element 110 is preferably obviated and a luer is integrally made with the syringe barrel 104, whereas the forward stopper is releasably connected to the luer in storage in order to seal the first substance and is configured to be released from the luer of the syringe during use.

It is noted that the first and the second substances may be in a form of solid medicament preparation, powder or liquid.

The syringe barrel 104 is preferably made of glass. The syringe barrel has a generally cylindrical shape and extends along the longitudinal axis 103. A bypass protrusion 128 is disposed generally at an intermediate location of the syringe barrel 104 along axis 103. The bypass protrusion 128 generally extends radially outwardly from an outer surface of the syringe barrel 104 to facilitate fluid passage between the two chambers formed within the syringe barrel 104 between each pair of pistons.

It is appreciated that syringe assembly 102 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

A plunger rod assembly 130 preferably includes a plunger rod inner portion 132 and a plunger rod outer portion 134, which are preferably fixedly coupled with each other upon insertion of the plunger rod inner portion 132 into the plunger rod outer portion 134. Alternatively, the plunger rod assembly 130 may be an integrally formed element or split in any other way suitable for manufacturing.

The plunger rod assembly 130 is operatively associated with the finger grip 120 and is displaceable with respect thereto and thus, with respect to the syringe barrel 104. In certain operative orientations, the plunger rod assembly 130 is rotatable about longitudinal axis 103 with respect to the finger grip 120 and the syringe barrel 104. In other operative orientations, the plunger rod assembly 130 is axially displaceable along longitudinal axis 103 relative to said finger grip 120 and the syringe barrel 104.

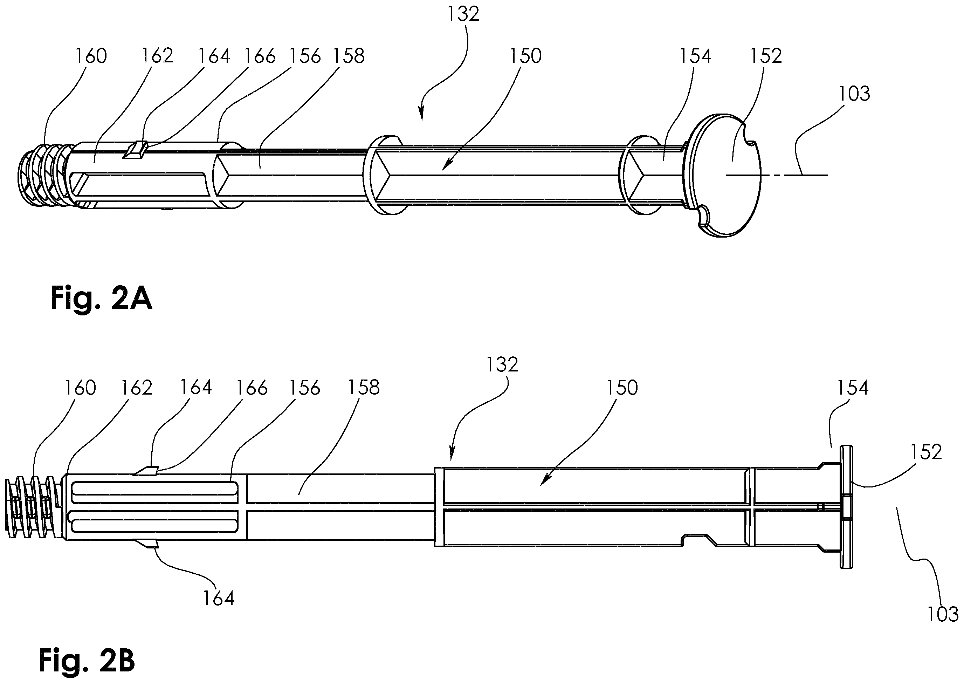

Reference is now made to Figs. 2A and 2B, which are respectively a simplified perspective view and planar side view of the plunger rod inner portion 132 forming part of the dual chamber syringe 100 of Figs. 1A & 1B.

The plunger rod inner portion 132 preferably is an integrally formed element, preferably injection molded of plastic and is arranged along longitudinal axis of symmetry 103.

The plunger rod inner portion 132 preferably includes a longitudinal shaft 150 terminating at a generally circular flange 152 at a rearward end 154 thereof. The circular flange 152 extends generally radially outwardly from the outer surface of the longitudinal shaft 150 and is disposed generally transversely with respect thereto. A generally widened longitudinal portion 156 extends forwardly from a forward end 158 of the longitudinal shaft 150 and an externally threaded protrusion 160 extends forwardly from a forward end 162 of the widened longitudinal portion 156.

Typically, two diametrically opposed snap protrusions 164 are formed on two opposite sides of the widened longitudinal portion 156. Snap protrusions 164 generally extend radially outwardly from the widened longitudinal portion156 and are adapted for fixedly connecting the plunger rod inner portion 132 with the plunger rod outer portion 134. Each of the snap protrusions 164 preferably includes a rearwardly facing surface 166, which is disposed generally transversely with respect to the longitudinal axis 103.

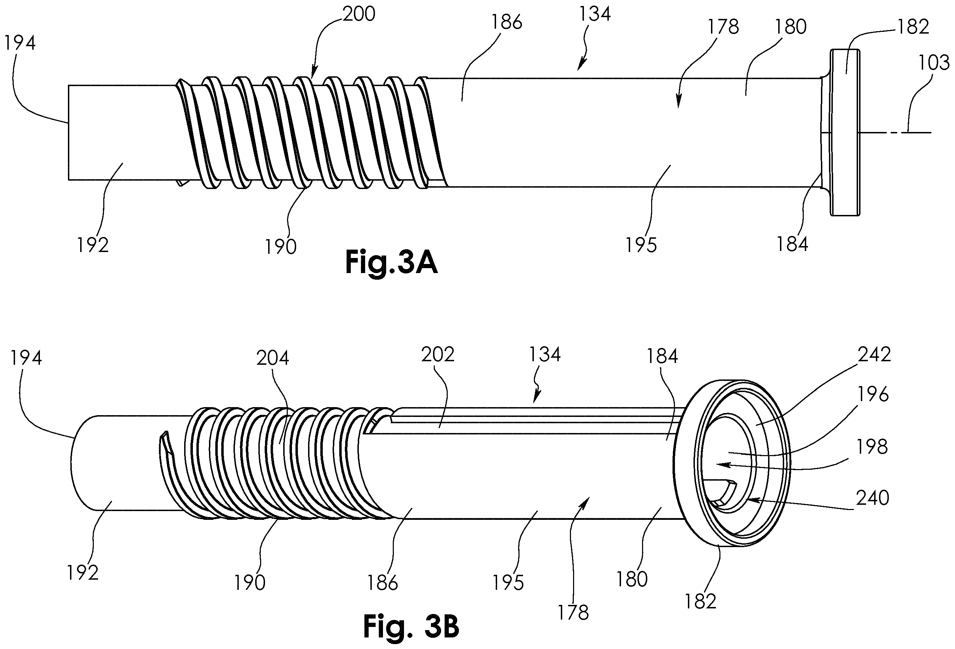

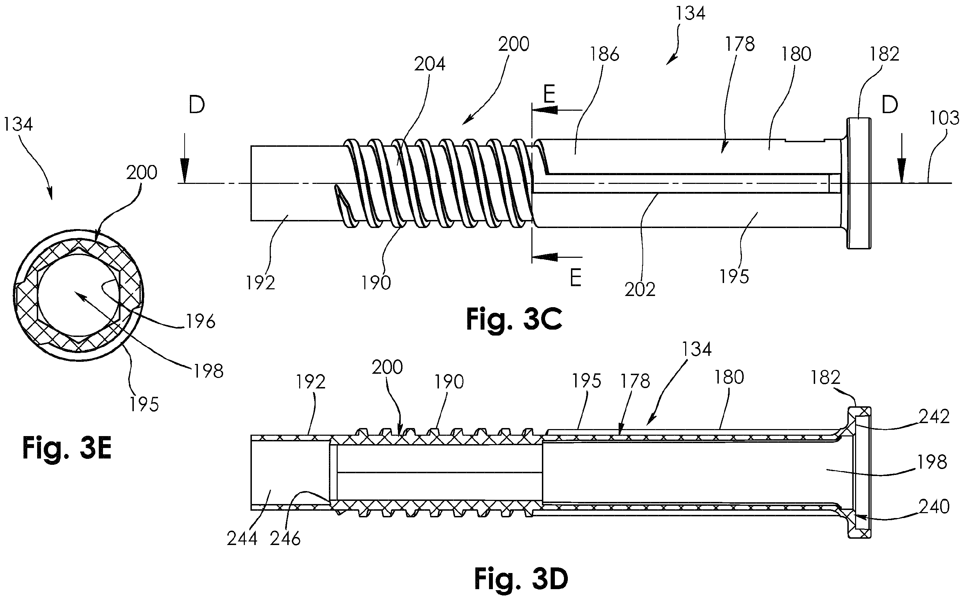

Reference is now made to Figs. 3A, 3B, 3C, 3D and 3E, which are respectively a simplified perspective view, two simplified plan side views and two simplified sectional views taken along lines D - D and E - E in Fig. 3C of the plunger rod outer portion 134 forming part of the dual chamber syringe 100 of Figs. 1A & 1B.

The plunger rod outer portion 134 preferably is an integrally formed generally cylindrical hollow element, preferably injection molded of plastic and is arranged along longitudinal axis of symmetry 103.

The plunger rod outer portion 134 has a longitudinal shaft 178, which preferably includes a longitudinal rearward portion 180, terminating at a generally circular flange 182 at a rearward end 184 thereof. The circular flange 182 extends generally radially outwardly from the outer surface of the rearward portion 180 and is disposed generally transversely with respect thereto. The rearward portion 180 has a forward end 186, from which an intermediate externally threaded portion 190 extends forwardly up to a forward portion 192, terminating at a forwardmost circumferential edge 194. It is noted that the outer diameter of the rearward portion 180 is somewhat greater than the outer diameter of the forward portion 192.

The plunger rod outer portion 134 has an outer surface 195 and an inner surface 196, formed by a longitudinal through bore 198.

A guiding track 200 is formed on the outer surface 195 of the plunger rod outer portion 134. The guiding track 200 includes a longitudinal track portion 202 and a helical track portion 204 connected thereto.

It is seen in Figs. 3B and 3D that an internal socket 240 is formed within circular flange 182, having a rearwardly facing surface 242 and adapted for receiving circular flange 152 of the plunger rod inner portion 132 and supporting thereof for preventing forward axial displacement of the plunger rod inner portion 132 relative to the plunger rod outer portion 134. The internal socket 240 communicated with the longitudinal through bore 198, which terminates at a forward bore portion 244, having a slightly greater diameter than through bore 198, thus forming a forwardly facing shoulder 246 between the two bores. The forwardly facing shoulder 246 is adapted for engaging snap protrusions 164 of the plunger rod inner portion 132 and thus prevent rearward axial displacement of the plunger rod inner portion 132 relative to the plunger rod outer portion 134.

It is appreciated that alternatively, the engaging snap protrusion 164 might be located on the plunger rod outer portion 134 while engaging with a forwardly facing shoulder located on the plunger rod inner portion 132, thus preventing the plunger rod inner portion 132 from rearward axial displacement relative to the plunger rod outer portion 134.

It is appreciated that alternatively the plunger rod assembly 130 may be formed as a single integrally made element, without fixedly connecting plunger rod inner and outer portions.

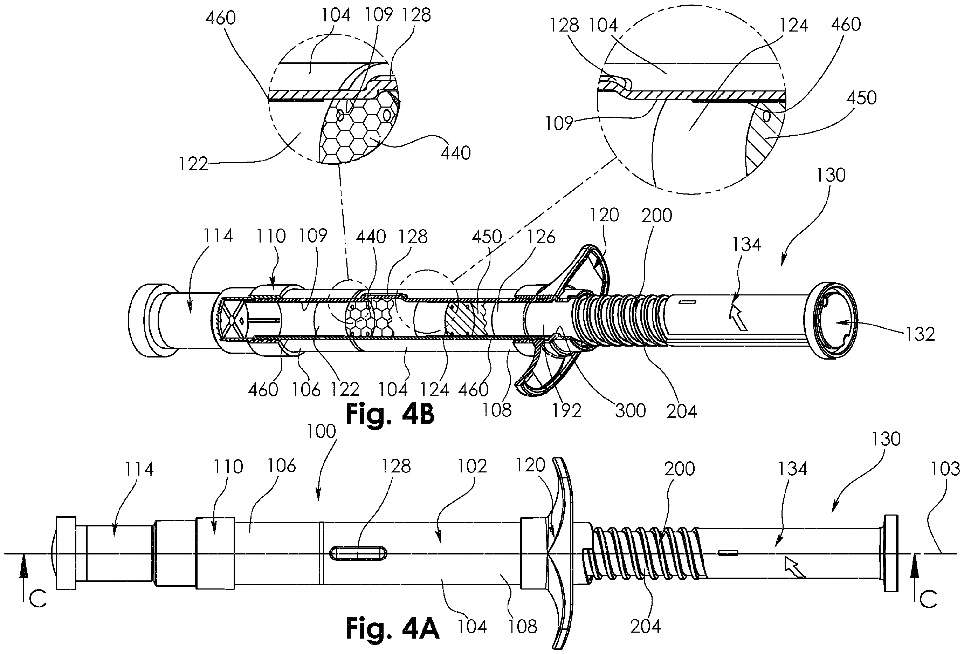

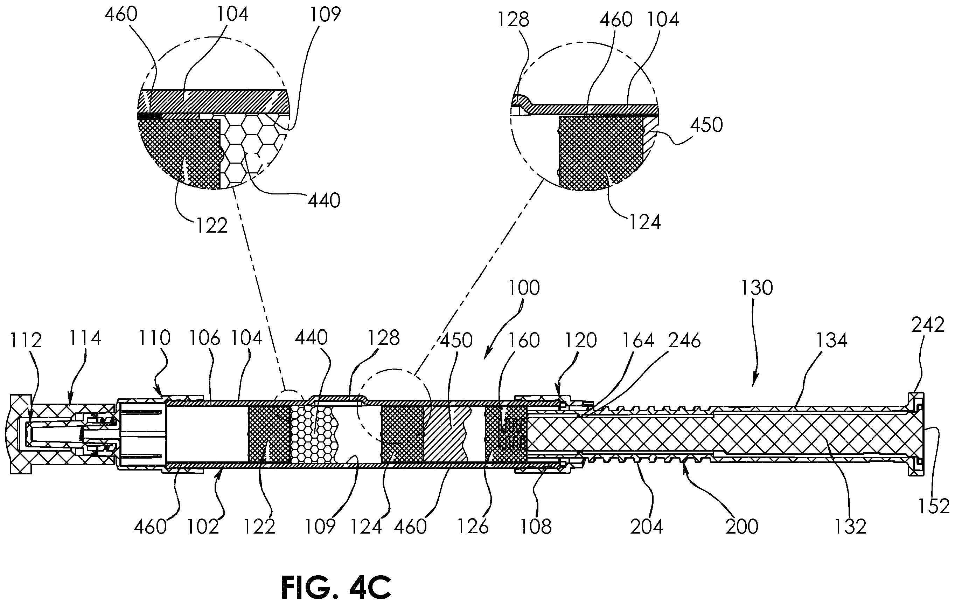

Reference is now made to Figs. 4A, 4B and 4C, which are simplified drawings of the dual chamber syringe 100 of Figs. 1A - 3E in a storage operative orientation, including respectively a simplified planar view, a partially cut-out perspective view and a simplified sectional view taken along lines C - C in Fig. 4A.

It is seen in Figs. 4A - 4C that the dual chamber syringe 100 is arranged along the longitudinal axis 103. The finger grip 120 is fixedly attached to the rearward end 108 of the syringe barrel 104.

The luer lock element 110 is fixedly attached to the forward end 106 of the syringe barrel 104. It is noted that alternatively, the syringe barrel 104 and the luer lock element 110 may be formed as an integral part. Further alternatively, a needle can be fixedly attached to the luer lock element 110 or to a luer that is formed as part of the syringe barrel, in case the syringe barrel and the luer lock element are integrally formed.

The inner cap element 112 is releasably mounted over the luer lock element 110 and is releasably held therein. The outer cap element 114 surrounds the inner cap element 112 and is disposed in friction-fit engagement therewith.

Alternatively, in an embodiment that comprises only two pistons, the luer lock element 110 is obviated and a luer is integrally made with the syringe barrel 104, whereas the forward stopper 122 is releasably connected to the luer in this storage operative orientation and configured to seal the first substance within the syringe barrel 104.

It is additionally seen in Figs. 4B and 4C that the forward piston 122 is sealingly slidably disposed with respect to the inner surface 109 of the syringe barrel 104 and is located generally in proximity to the forward end 106 of the syringe barrel 104. The intermediate piston 124 is also sealingly slidably disposed with respect to the inner surface 109 of the syringe barrel 104 and is located generally between the rearward end 108 of the syringe barrel 104 and between the bypass protrusion 128.

It is noted that drug preparation 440 is confined between the forward piston 122 and the intermediate piston 124 and the forward piston 122 is used for sealing the drug preparation 440 between the two pistons 122 and 124.

It is particularly seen in Fig. 4C that the plunger rod assembly 130 is threadably coupled to the rearward piston 126 by means of threadable interconnection between externally threaded protrusion 160 of plunger rod inner portion 132 with the rearward piston 126.Alternatively, the plunger rod assembly 130 may be coupled to the rearward piston 126 by any other means, such as fastening, adhesion etc, or integrally made therewith.

It is noted that solvent 450 is confined between the rearward piston 126 and the intermediate piston 124.

The plunger rod inner portion 132 is inserted into the plunger rod outer portion 134 and is fixedly held therein, thereby forming the plunger rod assembly 130. It is noted that the flange 152 of the plunger rod inner portion 132 is supported against rearwardly facing surface 242 of the plunger rod outer portion 134 to prevent forward displacement of the plunger rod inner portion 132 relative to the plunger rod outer portion 134. It is further noted that the snap protrusions 164 of the plunger rod inner portion 132 are supported against forwardly facing shoulder 246 of the plunger rod outer portion 134 to prevent rearward displacement of the plunger rod inner portion 132 relative to the plunger rod outer portion 134.

The plunger rod inner portion 132 is preferably prevented from rotation relative to the plunger rod outer portion 134 due to the fact that the plunger rod inner portion 132 is received into a generally hexagonal bore 198 of the plunger rod outer portion 134, As particularly seen in Fig. 3E.

The plunger rod assembly 130 is operatively associated with the finger grip 120 and is displaceable with respect thereto and thus, with respect to the syringe barrel 104. As described in detail in U.S. Patent Application Serial No. 16/994,596, the finger grip 120 comprises at least one tooth 300 protruding radially inwardly from an inner surface thereof and configured to engage the guiding track 200 of the plunger rod outer portion 134.

In this storage operative orientation, the plunger rod assembly 130 is partially axially inserted into the syringe barrel 104, such that the teeth 300 of the finger grip 120 are not yet engaged with the guiding track 200 of the plunger rod outer portion 134.

It is particularly seen in Fig. 4B that the teeth 300 of finger grip 120 are disposed along the longitudinal extent of the forward portion 192 of the plunger rod outer portion 134 and are axially forwardly spaced with respect to the helical track portion 204 of the guiding track 200.

It is a particular feature of an embodiment of the present invention that only a portion of the inner surface 109 of the syringe barrel 104 is treated with friction reducing agent 460.

It is a further particular feature of an embodiment of the present invention that only a portion of the inner surface 109 of the syringe barrel 104 is treated with friction reducing agent 460, preferably the annular portion of the inner surface 109 that contains the bypass protrusion 128 and the area adjacent the bypass protrusion 128 is not treated with friction reducing agent 460. Specifically, the inner surface 109 of the syringe barrel 104 is only treated with friction reducing agent 460 in the region where the intermediate piston 124 is configured to be seated in storage and axially rearwardly therefrom and in the region where the forward piston 122 is configured to be seated in storage and axially forwardly therefrom. The inner surface 109 of the syringe barrel 104 defined between the forward piston 122 and the intermediate piston 124 is not treated with friction reducing agent 460, thus preventing contact between one substance, such as drug preparation 440, and between the friction reducing agent 460 in this storage operative orientation.

It is particularly seen in Figs. 4B and 4C that preferably a forward portion of the forward piston 122 and a rearward portion of the intermediate piston 124 contact the areas of the inner surface 109 that is treated with friction reducing agent 460 in this storage operative orientation. Preferably, the entire circumference of the rearward piston 126 contacts one of the areas of the inner surface 109 that are treated with friction reducing agent 460.

It is noted that treatment of the inner surface 109 of the syringe barrel 104 with friction reducing agent 460 reduces friction forces that are created during displacement of the plunger rod assembly 130 and the pistons 122, 124 and 126 relative to the syringe barrel 104.

It is a particular feature of an embodiment of the present invention that partial treatment of the inner surface 109 with friction reducing agent 460 provides both for reduced friction forces and avoiding damage or agglomeration of the substance, such as drug preparation 440, during storage, due to undesirable contact of the substance with friction reducing agent 460 for prolonged periods of time.

It is a particular feature of an embodiment of the present invention that two chambers are formed within the dual chamber syringe 100, whereas one substance, such as drug preparation 440, is contained within the first chamber defined between the forward piston 122 and the intermediate piston 124 and the second substance, such as solvent 450, is contained within the second chamber defined between the intermediate piston 124 and the rearward piston 126, whereas the inner surface 109 of the syringe barrel 104 defining the first chamber is not treated with friction reducing agent 460 and the remainder of the inner surface 109 is treated with friction reducing agent 460.

It is noted that various materials can be used as friction reducing agents 460 and for pistons 122, 124 and 126. For example, a grade of friction reducing agent can be used, which may be cured to create a solid-state friction reducing agent layer, which prevents leakage of the friction reducing agent 460 into the first chamber and thus prevents its contact with the drug preparation 440. Furthermore, partial coating or partial lubrication of the inner surface 109 of the syringe barrel 104 may be used in order to reduce friction forces between the pistons 122, 124 and 126 during displacement of the plunger rod 130 relative to the syringe barrel 104.

The friction reducing agent 460 may be a lubricant, such as silicone, for example.

It is noted that alternatively a standard plunger rod can be utilized in accordance with an embodiment of the present invention instead of the plunger rod assembly 130.

In an alternative embodiment mentioned hereinabove, only two pistons may be disposed within the syringe barrel 104, such as first piston 124 and second piston 126. The two pistons 124 and 126 are configured to be slidably displaceable relative to the syringe barrel 104 and a forward stopper 122 may be releasably connected to the forward end of the syringe barrel 104 or to the luer thereof. In accordance with this embodiment of the present invention the portion of the inner surface 109 that is not treated with the friction reducing agent 460 is disposed between the first piston 124 and the forward stopper 122 and the remainder inner surface 109 of the barrel 104 is treated with the friction reducing agent 460. The first substance is contained within the first chamber, which is defined between the forward stopper 122 and the first piston 124, whereas the first substance is typically a drug preparation. The second substance is contained within the second chamber, which is defined between the first piston 124 and the second piston 126, whereas the second substance is typically a solvent. The inner surface defined by the first substance chamber is preferably not treated with friction reducing agent 460 to avoid contact between the medication and the friction reducing agent 460, but the remainder of the inner surface of the barrel 104 is treated with friction reducing agent 460. This partial treatment of the inner surface of the barrel allows for avoiding negative effects resulting from contact between the medication and the friction reducing agent 460 in storage and providing reduced friction forces while displacing the pistons relative to the barrel 104.

It is further noted that a single chamber medical container may be utilized in accordance with an embodiment of the present invention, such as a standard prefilled syringe or cartridge, which contains medication concealed therewithin by a piston. It is a particular feature of an embodiment of the present invention that the medication is concealed within a substance chamber defining an inner surface. The inner surface defined by this substance chamber is preferably not treated with friction reducing agent 460 to avoid contact between the medication and the friction reducing agent 460, but the remainder of the inner surface of the syringe or cartridge is treated with friction reducing agent 460. This partial treatment of the inner surface of the syringe or cartridge allows for avoiding negative effects resulting from contact between the medication and the friction reducing agent 460 in storage and providing reduced friction forces while displacing a plunger rod rearwardly relative to the syringe or cartridge, such as during aspiration of fluid into the substance chamber.

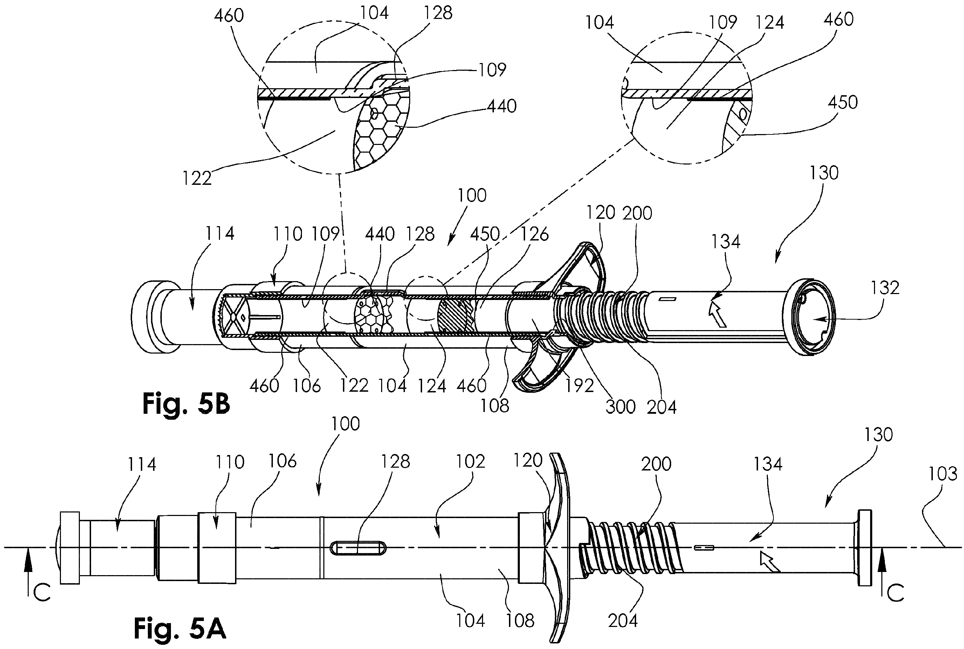

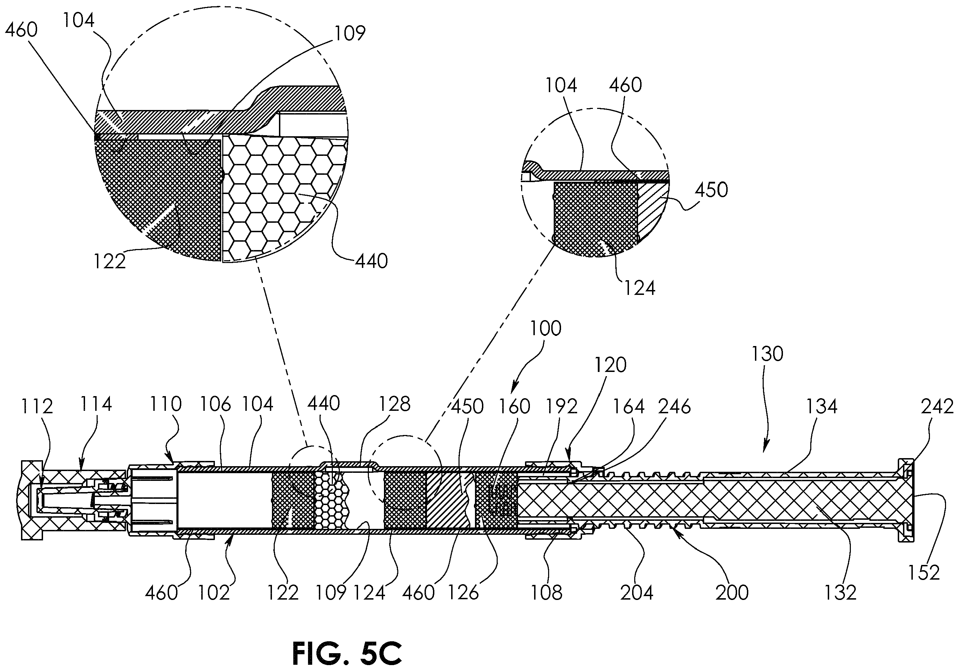

Reference is now made to Figs. 5A, 5B and 5C, which are simplified drawings of the dual chamber syringe 100 of Figs. 1A - 3E in a pre-reconstitution operative orientation, including respectively a simplified planar view, a partially cut-out perspective view and a simplified sectional view taken along lines C - C in Fig. 5A.

It is seen in Figs. 5A - 5C that the plunger rod assembly 130 is preferably slightly axially forwardly displaced relative to the syringe barrel 104 along longitudinal axis 103 to compensate for dimension variations between the different components of the dual chamber syringe 100 and to compensate for piston displacement due to pressure variations within the syringe assembly 102, such as during shipping, for example. It is appreciated that the axial displacement of the plunger rod assembly 130 relative to the syringe barrel 104 may be minimal in this operative orientation.

It is appreciated that all spatial relationships between the various components of the dual chamber syringe 100 remain the same as described hereinabove with respect to the storage operative orientation illustrated in Figs. 4A - 4C, besides the following spatial relationships:
It is particularly seen in Fig. 5B that in this pre-reconstitution operative orientation, the plunger rod assembly 130 is forwardly axially displaced up to engagement of the teeth 300 of finger grip 120 with the helical track portion 204 of the guiding track 200.

It is also seen in Fig. 5B and 5C that the rearward piston 126 is forwardly axially displaced relative to the syringe barrel 104 along with the plunger rod assembly 130, due to the threadable engagement therebetween. The intermediate piston 124 and the forward piston 122 are correspondingly axially forwardly displaced relative to the syringe barrel 104 due to the hydraulic pressure created in the second syringe chamber containing the solvent 450 and in the first syringe chamber containing the drug preparation 440. It is noted that in this pre-reconstitution operative orientation, the intermediate piston 124 is still rearwardly spaced from the bypass protrusion 128 of the syringe barrel 104.

It is noted that friction forces between the pistons 122, 124 and 126 and the inner surface 109 of the syringe barrel 104 are reduced during displacement of the plunger rod assembly 130 relative to the syringe barrel 104 due to the partial treatment of the inner surface 109 with friction reducing agent 460. In this exemplary embodiment, the lack of contact between the first substance contained in the first chamber of the dual chamber syringe 100 and the friction reducing agent 460 is maintained. Particularly, as seen in Figs. 5B and 5C, the drug preparation 440 contained within the first chamber of the dual chamber syringe 100 does not contact the areas of the inner surface 109 that are treated with friction reducing agent 460 in this pre-reconstitution operative orientation. Alternatively, the drug preparation 440 may contact the friction reducing agent 460 in this pre-constitution operative orientation, since this operative orientation is momentary and thus does not cause damage to the drug preparation 440.

It is particularly seen in Figs. 5B and 5C that preferably only a rearward portion of the intermediate piston 124 and only a forward portion of the forward piston 122 contact the area of the inner surface 109 that is treated with friction reducing agent 460 in this pre-reconstitution operative orientation, due to the slight axial displacement of the plunger rod assembly 130 relative to the syringe barrel 104. Preferably, the entire circumference of the rearward piston 126 contacts the area of the inner surface 109 that is treated with friction reducing agent 460.

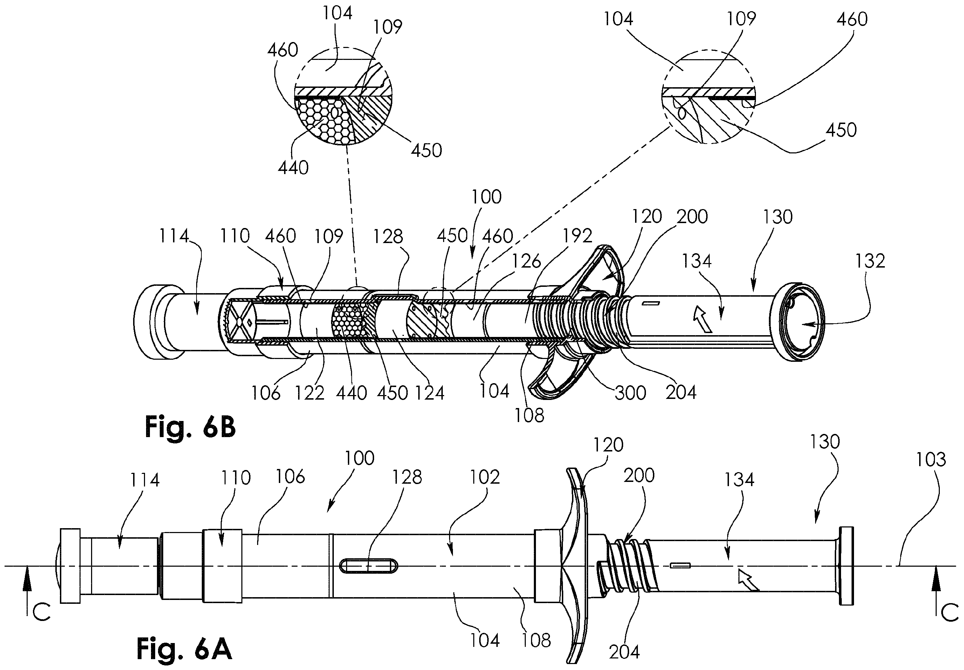

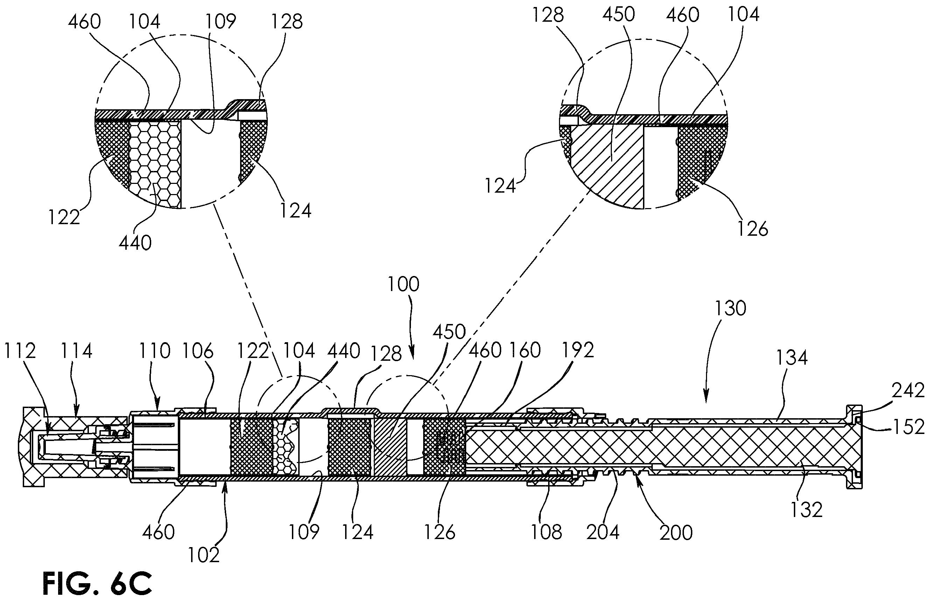

Reference is now made to Figs. 6A, 6B and 6C, which are simplified drawings of the dual chamber syringe 100 of Figs. 1A - 3E in a medicament reconstitution operative orientation, including respectively a simplified perspective view, a partially cut-out perspective view and a simplified sectional view taken along lines C - C in Fig. 6A.

It is seen in Figs. 6A - 6C that the plunger rod assembly 130 is axially forwardly advanced relative to the syringe barrel 104 by means of thread-like rotation of the plunger rod assembly 130 relative to the finger grip element 120. The plunger rod assembly 130 is rotated relative to the finger grip element 120 and thus relative to syringe barrel 104 about longitudinal axis 103 in the rotational direction and thereby longitudinally displaced forwardly along longitudinal axis 103 to provide for passage of solvent 450 into the chamber containing the drug preparation 440 through the bypass protrusion 128 of the syringe barrel 104 in order to reconstitute the drug preparation resulting in a liquid medicament solution 470 contained between the forward piston 122 and the intermediate piston 124.

The dual chamber syringe 100 is shown during the medicament reconstitution in Figs. 6A - 6C, whereas only a portion of the solvent 450 passed into the chamber containing the drug preparation 440 through the bypass protrusion 128.

It is noted that the longitudinal dimension of the externally threaded portion 190 is preferably defined as the length that is required to axially displace the intermediate piston 124 toward the bypass protrusion 128 of the syringe barrel 104 and to axially displace the rearward piston 126 up to engagement with the intermediate piston 124.

It is appreciated that all spatial relationships between the various components of the dual chamber syringe 100 remain the same as described hereinabove with respect to the pre-reconstitution operative orientation illustrated in Figs. 6A - 6C, besides the following spatial relationships:
In this medicament reconstitution operative orientation, the plunger rod assembly 130 is rotated about the longitudinal axis and due to thread-like engagement of the teeth 300 of finger grip 120 with the helical track portion 204 of the guiding track 200 of the plunger rod assembly 130, the plunger rod assembly 130 is forwardly displaced longitudinally along longitudinal axis 103.

It is noted that in this medicament reconstitution operative orientation shown in Figs. 6A - 6C, the plunger rod assembly 130 completed only a portion of its forward longitudinal displacement required for complete medicament reconstitution. It is specifically seen that the teeth 300 of the finger grip element 120 are disposed at an intermediate location along the helical track portion 204 of the guiding track 200.

It is seen in Figs. 6B and 6C that the rearward piston 126 is forwardly axially displaced relative to the syringe barrel 104 along with the forward displacement of the plunger rod assembly 130, due to the threadable engagement therebetween.

The rotational displacement of the plunger rod assembly 130 is translated to axial forward displacement thereof along longitudinal axis 103 due to thread-like engagement between the plunger rod assembly 130 and the finger grip element 120, specifically between the helical track portion 204 of the plunger rod assembly 130 and the teeth 300 of the finger grip element 120.

It is noted that upon initiation of axial forward displacement of the plunger rod assembly 130, all three pistons, namely the forward piston 122, the intermediate piston 124 and the rearward piston 126 are displaced together and remain at a constant distance one from another up until the point where the intermediate piston 124 is axially aligned with the bypass protrusion 128 of the syringe barrel 104. At this point, when the intermediate piston 124 is aligned with the bypass protrusion 128, fluid flow passage is established between the two chambers of the dual chamber syringe 100 and solvent 450 is transferred into the chamber containing the drug preparation 440 through the bypass protrusion 128.

Once the intermediate piston 124 is aligned with the bypass protrusion 128, the plunger rod assembly 130 along with the rearward piston 126 are forwardly displaced axially due to the thread-like engagement that is explained in detail hereinabove, toward the intermediate piston 124 until all solvent 450 is transferred into the chamber containing drug preparation 440.

It is noted that in the medicament reconstitution operative orientation, shown in Figs. 6A - 6C, the friction forces between the pistons 122, 124 and 126 and the inner surface 109 of the syringe barrel 104 are reduced during displacement of the plunger rod assembly 130 relative to the syringe barrel 104 due to the partial treatment of the inner surface 109 with friction reducing agent 460. Particularly, as seen in Figs. 6B and 6C, the drug preparation 440 contained within the first chamber of the dual chamber syringe 100 now contacts the inner surface 109 that is treated with friction reducing agent 460 in this medicament reconstitution operative orientation, but since this operative orientation is momentary, this contact does not cause damage to the drug preparation 440.

It is particularly seen in Figs. 6B and 6C that preferably substantially the entire outer surface of the rearward piston 126 and substantially the entire outer surface of the forward piston 122 contact the area of the inner surface 109 that is treated with friction reducing agent 460 in this medicament reconstitution operative orientation. It is noted that the outer surface of the intermediate piston 124 does not contact the inner surface 109 that is treated with friction reducing agent 460.

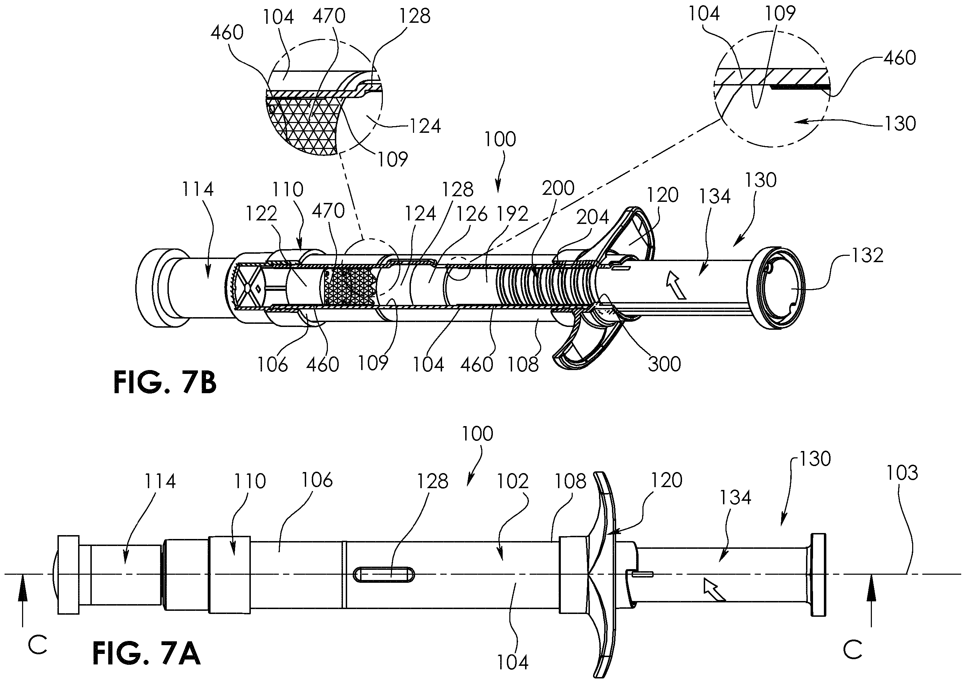

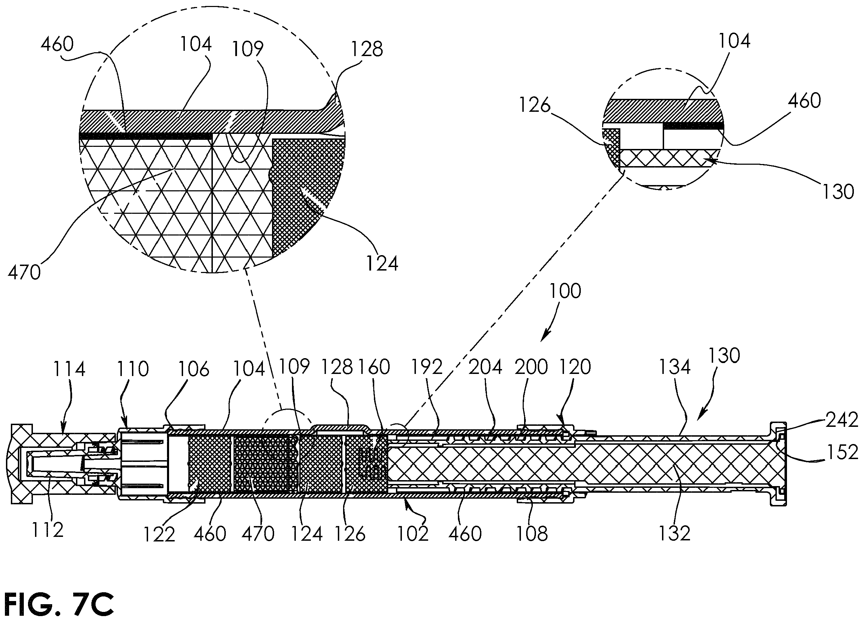

Reference is now made to Figs. 7A, 7B and 7C, which are simplified drawings of the dual chamber syringe 100 of Figs. 1A - 3E in an end of medicament reconstitution operative orientation, including respectively a simplified perspective view, a partially cut-out perspective view and a simplified sectional view taken along lines C - C in Fig. 7A.

It is seen in Figs. 7A - 7C that the plunger rod assembly 130 is further axially forwardly advanced relative to the syringe barrel 104 by means of further thread-like rotation of the plunger rod assembly 130 relative to the finger grip element 120. The plunger rod assembly 130 is further rotated relative to the finger grip element 120 and thus relative to syringe barrel 104 about longitudinal axis 103 in the rotational direction indicated by arrow 250 and thereby longitudinally displaced forwardly along longitudinal axis 103 to provide for passage of solvent 450 into the chamber containing the drug preparation 440 through the bypass protrusion 128 of the syringe barrel 104 in order to reconstitute the drug preparation resulting in a liquid medicament solution 470 contained between the forward piston 122 and the intermediate piston 124.

The dual chamber syringe 100 is shown at the end of medicament reconstitution in Figs. 7A - 7C, whereas the entire amount of the solvent 450 passed into the chamber containing the drug preparation 440 through the bypass protrusion 128 and created liquid medicament solution 470 now contained between the forward piston 122 and the intermediate piston 124.

It is appreciated that all spatial relationships between the various components of the dual chamber syringe 100 remain the same as described hereinabove with respect to the reconstitution operative orientation illustrated in Figs. 6A - 6C, besides the following spatial relationships:
In this end of medicament reconstitution operative orientation, the plunger rod assembly 130 completed its thread-like rotation relative to the finger grip element 120 and now the teeth 300 of the finger grip element 120 engage the longitudinal track portion 202 of the guiding track 200 of the plunger rod assembly 130.

It is particularly seen that upon further thread-like rotation of the plunger rod assembly 130 relative to the finger grip element 120 the teeth 300 of the finger grip element 120 reach the rearward end of the helical track portion 204, then continuously pass through the longitudinal track portion 202 of the guiding track 200.

In this end of medicament reconstitution operative orientation, further rotation of the plunger rod assembly 130 relative to the finger grip element 120 is prevented, due to engagement of the teeth 300 of the finger grip element 120 with the longitudinal track portion 202 of the guiding track 200.

It is noted that the plunger rod assembly 130 is prevented from rotational displacement relative to the finger grip element 120 and relative to the syringe assembly 102 during the entire injection process due to engagement of the guided teeth 300 of the finger grip element 120 with the longitudinal track portions 202 of the plunger rod assembly 130 along the entire longitudinal extent of the longitudinal track portions 202.

It is seen that at this end of medicament reconstitution operative orientation shown in Figs. 7A - 7C, the plunger rod assembly 130 completed its entire thread-like rotational displacement for complete medicament reconstitution. It is specifically seen that the teeth 300 of the finger grip element 120 are now disposed at the forward end of the longitudinal track portion 202 of the guiding track 200.

It is seen in Figs. 7B and 7C that the rearward piston 126 now abuts the intermediate piston 124, which has now at least partially forwardly spaced from the bypass protrusion 128 and the liquid medicament solution 470 is contained between the intermediate piston 124 and the forward piston 122.

It is appreciated that the dual-chamber syringe 100 is now ready for injection of the liquid medicament solution 470 upon removal of the outer cap element 114, subsequent removal of the inner cap element 112 and connection of a needle to the luer lock element 110.

It is appreciated that upon axial forward displacement of the plunger rod assembly 130 relative to the syringe barrel 104, all pistons 122, 124 and 126 are displaced together up until the point where the forward piston 122 reaches the intermediate portion of the luer lock element 110 and then the rearward piston 126 along with the intermediate piston 124 are displaced forwardly relative to the forward piston 122 until all liquid medicament solution 470 is transferred into the male luer through grooves formed in the luer lock element 110.

It is noted that in this end of medicament reconstitution operative orientation, as shown in Figs. 7A - 7C, the friction forces between the pistons 122, 124 and 126 and the inner surface 109 of the syringe barrel 104 are reduced during displacement of the plunger rod assembly 130 relative to the syringe barrel 104 due to the partial treatment of the inner surface 109 with friction reducing agent 460.

The first substance, such as medicament preparation 440 is now fully reconstituted with the solvent 450 and the liquid medicament solution 470 is now sealingly contained between the forward piston 122 and the intermediate piston 124. It is noted that the liquid medicament solution 470 may contact the friction reducing agent 460, but since this operative orientation is momentary, this contact does not cause damage to the drug preparation 440.

It is particularly seen in Figs. 7B and 7C that preferably substantially the entire outer surface of the forward piston 122 contacts the area of the inner surface 109 that is treated with friction reducing agent 460 in this end of medicament reconstitution operative orientation. It is noted that the outer surface of the intermediate piston 124 and of the rearward piston 126 does not contact the inner surface 109 that is treated with friction reducing agent 460.

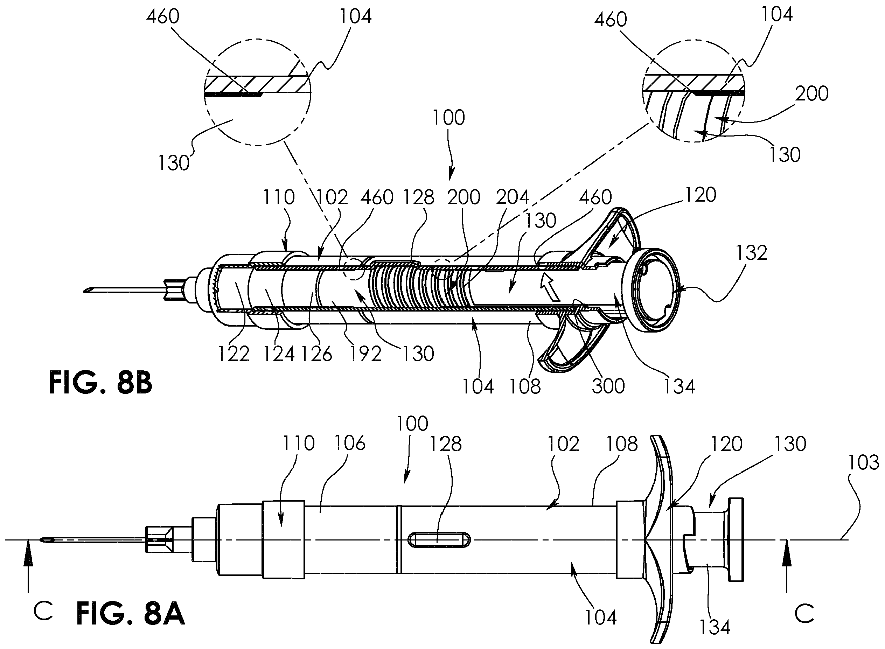

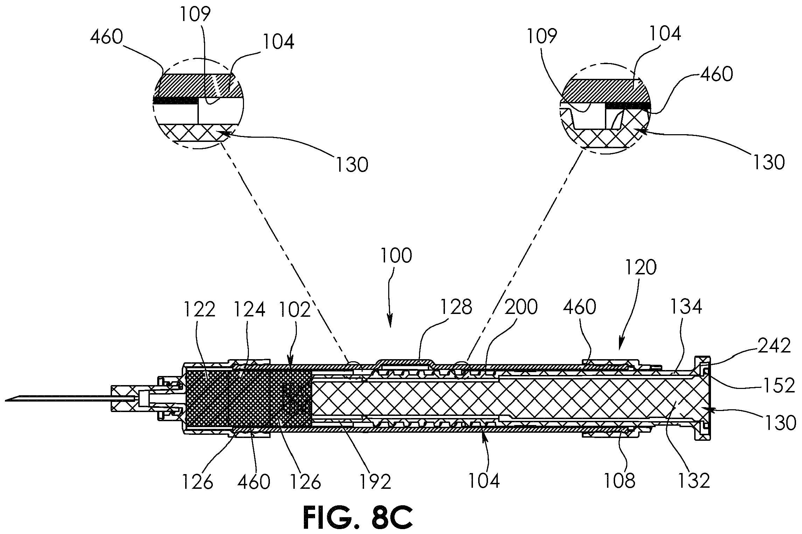

Reference is now made to Figs. 8A, 8B and 8C, which are simplified drawings of the dual chamber syringe 100 of Figs. 1A - 3E in an end of injection operative orientation, including respectively a simplified perspective view, a partially cut-out perspective view and a simplified sectional view taken along lines C - C in Fig. 8A.

It is seen in Figs. 8A - 8C that the plunger rod assembly 130 is axially forwardly displaced relative to the syringe barrel 104 by means of pushing the plunger rod assembly 130 axially along longitudinal axis 103 relative to the finger grip element 120 and thus relative to the syringe barrel 104 to provide for passage of liquid medicament solution 470 through the needle.

The dual chamber syringe 100 is shown at the end of injection in Figs. 8A -8C, whereas the entire amount of the liquid medicament solution 470 passed into the needle.

It is appreciated that all spatial relationships between the various components of the dual chamber syringe 100 remain the same as described hereinabove with respect to the end of reconstitution operative orientation illustrated in Figs. 7A - 7C, besides the following spatial relationships:
It is seen in Figs. 8A - 8C that during injection of liquid medicament solution 470, the plunger rod assembly 130 is forwardly displaced axially relative to the finger grip element 120 due to engagement of the teeth 300 of the finger grip element 120 with the longitudinal track portion 202 of the guiding track 200. It is particularly seen in Figs. 8B that the teeth 300 of the finger grip element 120 are now disposed adjacent the rearward end of the longitudinal track portion 202 of the guiding track 200.

It is seen in Figs. 8B and 8C that all three pistons 122, 124 and 126 are now disposed at their forwardmost position and abut each other, while all liquid medicament solution 470 is ejected from the dual chamber syringe 100.

It is a particular feature of an embodiment of the present invention that the friction forces between the pistons 122, 124 and 126 and the inner surface 109 of the syringe barrel 104 are reduced during displacement of the plunger rod assembly 130 relative to the syringe barrel 104 due to the partial treatment of the inner surface 109 with friction reducing agent 460. It is appreciated that during ejection of the liquid medicament solution 470 out of the syringe barrel 104, the outer surface of at least one of the pistons 122, 124 and 126 contacts the inner surface 109 of the syringe barrel 104 that is treated with friction reducing agent 460, thus minimizing the high friction forces that the user would otherwise have to overcome during displacement of the plunger rod 130 with all of the three pistons 122, 124 and 126 relative to the syringe barrel 104. Preferably, the outer surface of all three of the pistons 122, 124, 126 contacts the inner surface 109 of the syringe barrel 104 that is treated with friction reducing agent 460.

It will be appreciated by persons skilled in the art that the present invention is not limited by what has been particularly shown and described hereinabove. Rather the scope of the present invention includes both combinations and sub-combinations of various features described hereinabove as well as variations and modifications thereof which are not in the prior art.

## Claims

1. A medical container, comprising:
a barrel having an inner surface partially treated with a friction reducing agent;
the barrel having at least one substance chamber;
and wherein said at least one substance chamber defines a portion of said inner surface, which is not treated with said friction reducing agent.

2. The medical container according to claim 1, and wherein said at least one substance chamber defines two substance chambers and at least one of said two substance chambers defines said portion of said inner surface, which is not treated with said friction reducing agent.

3. The medical container according to claim 1 or 2, and wherein a drug preparation is configured to be contained within said at least one substance chamber confined by a piston, and the inner surface defined by said at least one substance chamber is not treated with friction reducing agent to avoid contact between the drug preparation and the friction reducing agent, and wherein the remainder of the inner surface of the barrel is treated with friction reducing agent.

4. The medical container according to any of the preceding claims, wherein said friction reducing agent is a lubricant.

5. The medical container according to any of the preceding claims, wherein said friction reducing agent is a coating substance.

6. The medical container according to claim 3, wherein partial treatment of the inner surface of the barrel with friction reducing agent provides both for reduced friction forces between the barrel and the piston and for avoiding damage of the drug preparation during storage, due to lack of contact between the treated area of the barrel and the drug preparation.

7. The medical container according to any of the preceding claims, and wherein said barrel having a forward end and a rearward end;
a forward stopper, a first piston and a second piston rearwardly spaced from said first piston; the first piston and the second piston are configured to be slidably displaceable relative to said barrel;
a first chamber is defined between said forward stopper and said first piston; a second chamber is defined between said first piston and said second piston;
and wherein said inner surface is partially treated with a friction reducing agent, such that in storage the inner surface defined by at least one of said first chamber and said second chamber is not treated with said friction reducing agent.

8. The medical container according to claim 7, wherein at least one bypass protrusion arranged along the longitudinal extent of said syringe barrel.

9. The medical container according to claim 7 or 8, wherein a plunger rod is configured to be partially inserted into said barrel and slidably displace at least one of said first and second pistons relative to said barrel.

10. The medical container according to any of claims 7 to 9, wherein a drug preparation is confined within the first chamber and a solvent is confined within the second chamber and upon longitudinal displacement of the first and second pistons relative to the barrel, the drug preparation and the solvent are configured for reconstitution and subsequent ejection from the barrel.

11. The medical container according to claim 8, wherein the inner surface that contains the bypass protrusion and adjacent to the bypass protrusion is not treated with friction reducing agent.

12. The medical container according to any of claims 7 to 11, wherein the inner surface of the barrel is only treated with friction reducing agent in the region where the first piston is configured to be seated in storage and axially rearwardly therefrom and in the region where the forward stopper is configured to be seated in storage and axially forwardly therefrom.

13. The medical container according to any of claims 7 to 12, wherein the inner surface of the barrel defined between the forward stopper and the first piston is not treated with friction reducing agent, thereby preventing contact between a drug preparation and between the friction reducing agent in storage.

14. The medical container according to claim 9, wherein partial treatment of the inner surface of the barrel with friction reducing agent is configured to reduce friction forces that are created during displacement of the plunger rod and the pistons relative to the barrel.

15. The medical container according to claim 10, wherein partial treatment of the inner surface of the barrel with friction reducing agent provides both for reduced friction forces and avoiding damage of the drug preparation during storage, due to lack of contact between the treated area and the drug preparation.
